Europäisches Patentamt

European Patent Office

Office européen des brevets

(11) Publication number: **0 427 399 A1**

# EUROPEAN PATENT APPLICATION

(21) Application number: 90311114.4

(22) Date of filing: 10.10.90

(51) Int. Cl.⁵: **C07K 15/06**, C12P 21/08,
C12N 5/20, C07K 3/28,
G01N 33/574, G01N 33/577

| | |
|---|---|
| The microorganism has been deposited with ATCC under number: HB 10251. | (71) Applicant: **MINNESOTA MINING AND MANUFACTURING COMPANY** **3M Center, P.O. Box 33427** **St. Paul, Minnesota 55133-3427(US)** |
| (30) Priority: **10.10.89 US 418674** | |
| (43) Date of publication of application: **15.05.91 Bulletin 91/20** | (72) Inventor: **The inventor has agreed to waive his entitlement to designation** |
| (84) Designated Contracting States: **CH DE FR GB IT LI NL SE** | (74) Representative: **Baillie, Iain Cameron et al** **c/o Ladas & Parry Isartorplatz 5** **W-8000 München 2(DE)** |

(54) Cancer marker and monoclonal antibodies specific therefor.

(57) Monoclonal antibodies specific for a cancer marker, as well as hybridoma cell lines producing such antibodies; a method of purifying the marker to an extent sufficient to use the marker for the production of such monoclonal antibodies; the purified marker itself; monoclonal antibodies prepared using the purified marker; and an assay for the marker using the monoclonal antibodies. The marker is one that: (1) exhibits RNA-releasing activity; (2) is detectable in the serum of substantially all patients having either disseminated or localized tumors by means of an assay for such RNA-releasing activity, and (3) exhibits a molecular weight of on the order of 70 kDa.

EP 0 427 399 A1

# CANCER MARKER AND MONOCLONAL ANTIBODIES SPECIFIC THEREFOR

## TECHNICAL FIELD

The present invention relates to the in vitro diagnosis of cancer. In another aspect this invention relates to proteins or other factors that are detectable in the blood of individuals harboring a cancer and not detectable in the blood of non-cancerous individuals, or that are detectable in significantly different quantities between cancerous and non-cancerous individuals. More particularly, the invention relates to the diagnosis of cancer by means of diagnostic immunoassays for the presence of such proteins.

## BACKGROUND OF THE INVENTION

The diagnosis of cancer using antibodies, including monoclonal antibodies, to detect markers that are diagnostic for cancer have been previously described. See, e.g., McIntire, K.R., "Tumor Markers: How Useful Are They?", Hosp. Pract., p. 55, Dec. 1984. Since many known cancer markers are useful only for a particular type or class of cancers, these markers tend to be relatively limited in application.

A cancer-associated marker protein is described in U.S. Pat. No. 4,746,539 (hereinafter the '539 patent). The marker of the '539 patent is described as being present in the serum of patients diagnosed as having a wide range of cancers, including localized tumors. This marker is described as being absent, or at least undetectable by means of the RNA-releasing assay described therein, in most if not all non-cancerous individuals. See, e.g., Schumm, et al., Cancer Res., 44:401-406 (1984). The marker is described as a partially purified protein preparation having a molecular weight of approximately 60,000 daltons (i.e., "60 kDa"). It appears from the data, e.g., FIG 1, of the '539 patent that the actual molecular weight of the protein preparation could be as high as approximately 70 kDa. Although, when present, this marker is detectable by the assay for RNA-releasing activity described in the '539 patent, such an assay is too burdensome and expensive for routine, large scale, effective clinical use.

The '539 patent describes the preparation of antisera to the claimed protein preparation and also postulates a method for the production of monoclonal antibodies to the protein preparation. It has been found however, as described by Applicant more fully below, that a protein preparation prepared as described in the '539 patent is not sufficiently pure for the preparation and selection of monoclonal antibodies specific for this marker.

It is recognized by those skilled in the art that an antigen must be of sufficient purity in order to be useful for the preparation of specific monoclonal antibodies. Were an impure antigen preparation to be injected into a suitable host, as an initial step in the typical preparation of monoclonal antibodies, the immune response of that host would include many antibodies made by the host against the impurities in the preparation rather than against the desired antigen itself. This non-antigen specific response hampers and can even prevent the preparation and selection of antigen-specific antibodies and corresponding hybrid cell lines.

A marker that is in certain respects similar to that described in the '539 patent is described in U.S. Pat. No. 4,725,538 to Senger. Senger's marker is distinguished from that of the '539 patent, inter alia, in that Senger's marker (1) is described as being present, albeit in lower levels, in normal human serum, and (2) is present at normal levels in the serum of patients carrying localized (i.e., non-disseminated) tumors. Senger describes partially purified protein preparations as well as antisera thereto, and also postulates a method for making monoclonal antibodies using such preparations.

## SUMMARY OF THE INVENTION

The present invention provides monoclonal antibodies exhibiting specificity for a cancer marker, as well as hybridoma cell lines producing such antibodies; a method of purifying the marker to an extent sufficient to use the marker for the production of such monoclonal antibodies; the purified marker itself; monoclonal antibodies that are prepared using the purified marker; and an assay for the marker using the monoclonal antibodies of the invention.

The present invention provides monoclonal antibodies specific for the cancer marker described in the '539 patent above, i.e., the marker being a protein that: (1) exhibits RNA-releasing activity, (2) is detectable in the serum of substantially all patients having either disseminated or localized tumors by means of an assay for such RNA-releasing activity, and is not detectable by means of such an assay in normal (i.e., non-cancer bearing) human serum, and (3) exhibits a molecular weight of on the order of 70 kDa.

In order to be used for the preparation of monoclonal antibodies the marker preferably is purified by a method that includes concentration of a marker-containing sample by hollow fiber filtration, followed by purification of the marker by chromatofocusing and then hydroxyapatite fractionation. The present marker is purified to an extent suitable to allow it to be used for the preparation of monoclonal antibodies, i.e., when injected into a suitable host the purified marker of the present invention elicits an immune response of suitable specificity to allow the fusion and selection of monoclonal antibodies specific for the marker.

## DETAILED DESCRIPTION

The word "marker", as used herein with respect to the instant invention, shall refer to a protein that: (1) exhibits RNA-releasing activity, (2) is detectable in the serum of substantially all patients having either disseminated or localized tumors by means of an assay for such RNA-releasing activity, and is not detectable by means of such an assay in normal (i.e., non-cancer bearing) human serum, and (3) exhibits a molecular weight of on the order of 70 kDa.

The term "RNA-releasing activity", as used herein, refers to the biochemical assay for RNA transport activity described in the '539 patent, the entire disclosure of which is hereby incorporated by reference.

The purification of marker from a sample preferably involves the steps of concentration followed by protein purification. Optionally other steps may be used before, during, between, or after concentration or purification of marker, including but not limited to such preliminary clarification steps as centrifugation and/or filtration in order to remove cells and particulate matter. Samples can generally be frozen after such preliminary steps, for later use. Optionally one can include steps after the purification of marker, e.g., to obtain portions or alternative forms of the marker itself.

Purification of the present marker can be accomplished using any suitable source. Suitable sources exhibit an optimal combination of such properties as yield, volume, stability, availability, and cost. Examples of suitable sources include, but are not limited to, the serum of humans harboring a cancer and the medium of human cancer cells grown in culture.

Preferred sources of the present marker exhibit an optimal combination of such properties as concentration of marker compared to the concentration of other proteins and contaminants. An example of a preferred source is the medium of human cancer cells grown in tissue culture. A particularly preferred source is the tissue culture medium of a human lymphoma cell line ("CEM"), which cell line produces a marker that appears to be related to the human marker described in the '539 patent, inter alia, on the basis of RNA-releasing activity, molecular weight, and isoelectric point.

A preferred technique for the concentration of a sample containing marker is the use of hollow fiber filtration. Immunoaffinity can also be used, and is a preferred concentrating technique in situations where one or more specific antibodies is available in sufficient amounts and with sufficient affinity for such purposes.

The preferred concentration technique of hollow fiber filtration can be used in any suitable manner. See, generally, Wehr, J. Chromatog., 418:27-50 (1987), the disclosure of which is hereby incorporated by reference. Proper selection of the porosity of the filtration unit can significantly reduce the total amount of unwanted protein remaining. Using cell-conditioned medium as a sample, it was found preferable to rapidly concentrate the medium using porous hollow fibers having a molecular cutoff of nominally 10 kDa. Since marker is on the order of 70 kDa, the marker was fully retained while water and low molecular weight nutrients and cell products were removed. Fifty-fold or greater concentration was not uncommon when starting with dilute samples.

Samples are preferably concentrated to the point where further concentration is technically limited, e.g., by limitations inherent in the concentrating method or device, and or by the solubility limit of protein in the sample. Concentrating cell-conditioned medium with a hollow fiber filtration device as described herein for instance, on the order of 50-fold concentration is generally achievable.

Following concentration, samples are subjected to protein purification in order to obtain a preparation of sufficient purity to prepare monoclonal antibodies. After repeated attempts using a wide variety of purification techniques and conditions, Applicant was able to sufficiently purify the marker of the instant invention using a particular approach, namely chromatofocusing followed by hydroxyapatite chromatography.

Isoelectric focusing (i.e., separation based on differences in pI) has been developed in the art into a preparative chromatography method called chromatofocusing. See generally, Sluyterman, et al., J. Chromatog., 150:17-44 (1978), the disclosure of which is hereby incorporated by reference. The method is capable of high resolution while retaining a throughput capacity intermediate between the high levels of ion exchange and the low levels of size exclusion.

Chromatofocusing of concentrated marker between pH 6.3 and pH 4.0 using a Pharmacia Mono PTM column yielded 10 peaks of protein, four of which were between pH 5.5 and pH 5.0, the pI range of marker. Peaks 2 and 4 contained measurable RNA transport activity. Each of the two peaks also consisted of several protein bands as determined by electrophoretic analysis. Among these, peak 2 contained two 70 kDa bands, a major band at pI 5.7 and a minor one at pI 5.1. Peak 2 also consisted of several other minor bands, ranging from 5 kDa to 40 kDa. Peak 4 had a major 70 kDa band at pI 5.3 and a minor 60 kDa band at pI 4.8, as well as several minor bands which were larger than 100 kDa. There was no evidence of a major albumin band in the active fractions. These same results were reproduced upon scaling up.

The final step in the preferred purification is fractionation on an hydroxyapatite chromatography column (e.g., "HPHT", Bio-Rad). Hydroxyapatite (i.e., calcium phosphate) chromatography is a technique in which bound proteins are eluted by an increasing phosphate gradient, as described for instance by M.J. Gorbunoff, Meth. Enzymol., 117:370-380, 1985, the disclosure of which is hereby incorporated by reference. Although it is believed that proteins bind to hydroxyapatite through ionic interactions, the resulting elution profiles are usually different from those typically obtained with standard ion exchange supports. Protein is eluted by an increasing gradient of inorganic phosphate buffered near neutrality. Such fractionations of the two active peaks (2 and 4) derived from the chromatofocusing step removed the minor bands of molecular weight higher than 70 kDa and lower than 60 kDa. While these inactive materials eluted as a broad band at very low phosphate concentration, the active fraction from each sample eluted as a large, sharp, narrow band at 250 mM phosphate.

Several elution variations were attempted to promote resolution of the two proteins by either varying the conditions for the hydroxyapatite column, or adding a third column, e.g., a Poly FTM fluorocarbon column (DuPont). Among these variations were slower flow rate, shallower and narrower gradients, and changes in the pH and other constituents of the mobile phase. No condition improved the performance. Thus it appears that these proteins have very similar biochemical properties since they are inseparable chromatographically and extremely similar electrophoretically, and, perhaps, are each active in the RNA transport assay.

Various analytical procedures are available in order to determine the extent of purity of the marker after the above-described concentration and purification steps. Analytical two-dimensional gel electrophoresis for instance, is useful for determining, inter alia, the molecular weight of sample components. See generally, Andrews, A.T., Electrophoresis: Theory, Techniques, and Biochemical and Clinical Applications, 2nd ed., Oxford Univ. Press, (1986), the disclosure of which is incorporated herein by reference. Electrophoresis of the fractions showed that the active hydroxyapatite peak derived from peak 2 consisted of only two bands: the major one of 70 kDa with pI ranging from about 5.1 to about 5.7, and the minor one 60 kDa, pI 4.8. Similar treatment of the peak 4 material revealed that it consisted of a doublet band at 70 kDa, pI ranging from about 5.3 to about 5.7 and a 60 kDa band at pI 4.8.

Two-dimensional electrophoresis of peaks 2 and 4 combined revealed that the major band of peak 4 appeared to be identical to the minor band of peak 2 (60 kDa, pI 4.8). Size exclusion analysis run on samples of peak 2 after the above-described hydroxyapatite purification showed that most of the protein and all of the biologically active fractions eluted at 70 kDa, indicating that no further purification was achieved by size exclusion.

The peak 2 protein further purified on hydroxyapatite had the greatest total RNA-releasing activity as well as the highest RNA-releasing specific activity and was used for the preparation and selection of monoclonal antibodies.

The antibodies were prepared and selected using immunochemical laboratory techniques within the skill of those in the art. Five mice were injected with 100 μg each intraperitonealy, followed by an intraperitoneal booster injection 2 weeks later, and another booster (this one intravenous) three weeks after the first one and three days before the spleen was removed. Fusions were performed with lymphocytes from those mice which screened positive for cancer marker antibody using a sandwich bead ELISA assay with fluorometric detection. Cells were cultured in conventional hypoxanthine-aminopterin-thymidine ("HAT") selection media. Those that produced significant amounts of mouse IgG were cloned and subcloned. Selection of a clone was based on anti-marker activity using the sandwich ELISA and a Western blot. A positive blot was one in which mouse antibody bound to the 70 kDa region of the blotted sodium dodecyl sulfate ("SDS") polyacrylamide electrophoretogram of the marker. The 42 clones that were positive by these criteria were saved and expanded. These subclones were further screened against marker adsorbed on Immulon™ polystyrene microtiter wells (Dynatech Corp., Springfield, VA), resulting in 25 positives.

Interestingly, by the use of monoclonal antibodies prepared according to the present invention, it was shown that the human marker is in fact immunologically distinguishable from the rat marker in that monoclonal antibodies were prepared against the human marker that were not cross-reactive with the rat marker.

Monoclonals of the present invention are useful for a variety of purposes including diagnosis of cancer, evaluation of the effectiveness of cancer treatment, purification of marker, targeting marker in vivo, and immunologically characterizing marker within and between species and individuals. The preferred use of the monoclonal antibodies of the present invention is in the context of an assay for determining the presence of the marker in a clinical sample. Such an assay involves the the steps of

(a) providing a quantity of monoclonal antibody specific for marker,

(b) contacting the monoclonal antibody with a sample suspected of containing marker under conditions suitable to allow an immunochemical reaction to occur between antibody and marker,

(c) detecting the extent of such immunochemical reaction, and

(d) correlating the extent of immunochemical reaction with the presence or amount of marker present in the sample.

Such an assay can be performed in any of a number of suitable formats known to those skilled in the art, e.g., colorimetric, fluorometric, radiometric, chemiluminescent assays based on direct, indirect, or competitive assay formats.

The following EXAMPLES are provided to illustrate, but not limit, the scope of the invention. Unless otherwise indicated all percentages are by volume.

## EXAMPLES

## EXAMPLE 1

### Purification of Marker

"CEM" cells, an acute lymphoblastic T-cell leukemia cell line (American Type Culture Collection accession number ATCC CCL 119, also known as CCRF-CEM cells) which had been derived from a four-year old caucasian female (as described in G.E. Foley, et al., Cancer, 18:522-529 (1965) were used as a source of marker. Cells grown in spinner culture in "RPMI 1640" medium (Gibco, Grand Island, NY) supplemented with 1% (v/v) Nutridoma™ "HU" supplement (a serum-free protein supplement available from Boehringer-Mannheim, Indianapolis) and glutamine. Cell densities were maintained at $1 \times 10^6$ cells per ml.

The CEM cell-conditioned medium was dialyzed against 20 mM Tris, 1.0 M NaCl, pH 8.0, until the phenol red (the pH indicator from the cell growth medium) was removed, then against 5 mM Tris, pH 8.0. The sample (15 ml) was lyophilized and resuspended in 4 ml water.

1.5 ml of the resuspended sample was injected into an anion exchange column ("Mono Q" HPLC, 0.5 x 5 cm, Pharmacia, Piscataway, NJ) which was previously equilibrated with starting buffer (20 mM Tris, 20 mM NaCl, pH 8.0). After 4.0 ml of isocratic elution the separation was effected by an 18 ml linear NaCl gradient with the limit solution 500 mM NaCl in the same Tris buffer. Major protein bands (280 nm absorbance) were detected at 9.1 and 13.6 ml. 0.5 ml fractions were collected. Peak biological activity, as determined by the RNA transport assay as described in the '539 patent described earlier, was found in tube 21 (elution volume 10.5 ml).

Tubes 18-25 from the column were each separately fractionated on a size exclusion column ("Superose 12", 1 x 30 cm, Pharmacia) in 20 mM Tris, 200 mM NaCl, pH 8.0. All fractions contained a peak of protein eluting at 60-70 kDa (retention volume = 12.2 ml). The amount in any one fraction varied from 25-95% (as determined by absorbance at 280 nm) of the output protein (TABLE 1).

For purposes of TABLE 1 below, dialyzed, lyophilized CEM cell-conditioned medium was redissolved in 20 mM Tris, pH 8.0, injected into an anion exchange column and eluted by a linear gradient of NaCl (to 500 mM) as described above. Fractions eluting at 150-250 mM NaCl were individually fractionated on the size exclusion column. The relative amounts of protein eluting in the 60-70 kDa band (peak elution volume of 12.1 to 12.2 ml) as determined by a Pharmacia FPLC™ integrator are given below for each of the anion exchange fractions. Bovine serum albumin fractionates with a peak at 12.19 ml.

TABLE 1

| Relative Amounts of 60-70 kDa Protein in Anion Exchange Fractions of CEM Cell-conditioned Medium | | |
|---|---|---|
| Anion Exchange Fraction | Relative Area of 60-70 kDa peak | Retention Volume of 60-70 kDa (% of Total Area) |
| 18 | 18 | 12.22 ml (50%) |
| 19 | 93 | 12.21 (82) |
| 20 | 158 | 12.10 (95) |
| 21 | 109 | 12.14 (88) |
| 22 | 55 | 12.13 (73) |
| 23 | 30 | 12.11 (44) |
| 24 | 28 | 12.11 (33) |
| 25 | 22 | 12.15 (25) |

For purposes of determining RNA transport activity, anion exchange fraction samples were prepared as described above. One unit of RNA-releasing activity = % nuclear counts transported from 30 min pre-labeled nuclei during a 30 minute incubation in cell-free system according to the techniques described in Schumm, et al., Cancer Research, 33:1821-8 (1973), the disclosure of which is incorporated herein by reference. Briefly, during the course of the incubation of marker with radiolabeled rat liver nuclei, radioactive RNA from within the nucleus is transported into the medium. A basal rate of transport is observed if the incubation medium contains plasma from a cancer-free animal or patient. This basal rate increases two to three-fold when plasma from a tumor-bearing animal or patient is used. This experiment was designed to observe a decrease in the rate of transport caused by tumor plasma after incubation with an antibody against the cancer marker.

Biological (i.e., RNA transport) activity peaked in anion exchange fraction 21 and centered on size exclusion fractions 18 and 19, corresponding to 60-70 kDa (see TABLE 2 below).

TABLE 2

| RNA Transport Activity (Units/mg Protein) of each Anion Exchange Fraction after Size Exclusion Fractionation | | | | | | | | |
|---|---|---|---|---|---|---|---|---|
| Size Exclusion Fraction | Anion Exchange Fraction | | | | | | | |
| | 18 | 19 | 20 | 21 | 22 | 23 | 24 | 25 |
| 17 | 0 | 0 | 0.95 | 1.01 | 0 | 0 | 0 | 0 |
| 18 | 0.90 | 0.95 | 1.4 | 1.50 | 1.2 | 0.80 | 0 | 0 |
| 19 | 0.87 | 1.32 | 1.2 | 1.76 | 1.1 | 0.75 | 0.80 | 0 |
| 20 | 0 | 0 | 0.8 | 0.99 | 0 | 0 | 0 | 0 |

Fractions 17-19 from the size exclusion column were combined and dialyzed against 100 mM sodium phosphate, 1.28 M ammonium sulfate, pH 7.0. A hydrophobic interaction column ("Phenyl-Superose", 0.5 x 5 cm, Pharmacia) was equilibrated with the phosphate-sulfate buffer. The injected sample (500 μl) was isocratically eluted for 4 ml followed by a linear 10 ml descending gradient to 0.42 M ammonium sulfate in the original 100 mM phosphate buffer. A major protein eluted near the middle of the gradient (approximately 9.2 ml), coincident with the elution of albumin, as determined from control chromatograms of purified human albumin. However, bioactivity eluted earlier (approximately 2.0 ml), suggesting that the active protein is different from albumin.

EXAMPLE 2

DEAE-Silica Anion Exchange

The dialysis and Mono QTM anion exchange steps were replaced with a DEAE-silica HPLC column (Perkin-Elmer, Norwalk, Conn.). The column was developed by a linear 10 ml gradient from 25 to 500 mM NaCl in a buffer of 25 mM sodium phosphate, pH 6.0. Four major peaks of protein were eluted, two of which contained RNA transport activity, indicating that dialysis followed by Mono QTM anion exchange could replace the DEAE-silica HPLC column.

EXAMPLE 3

Silica Anion Exchange

The dialysis and Mono QTM anion exchange steps in EXAMPLE 2 were replaced by a 275 ml silica anion exchange column ("Accell", Waters Assoc., Medford, Mass.) packed in a 2.5 cm x 58 cm column (Pharmacia). This allows up to 1 liter volumes of CEM cell-conditioned medium to be processed at a time. The column and the conditioned medium were equilibrated in the pH 6.0 buffer. 500 ml of the medium was loaded onto the column through the buffer reservoir, followed by 500 ml isocratic elution using the starting buffer to elute the phenol red. Protein was eluted by a 25-512 mM NaCl gradient in the phosphate buffer (400 ml) and 512-1000 mM NaCl gradient (100 ml) followed by 300 ml of isocratic elution at 1000 mM NaCl. 25 mg of protein was recovered in the active region per liter of conditioned medium. This indicates that another anion exchange column could be used to purify the marker of the present invention.

EXAMPLE 4

Alternative Purification Method

In an attempt to determine the relationship of the marker of the present invention to albumin, phenol red was removed by chromatography on DEAE-cellulose equilibrated with 10 mM sodium phosphate, 50 mM NaCl, pH 6.0. 500 ml of CEM cell-conditioned medium (previously dialyzed against the pH 6.0 buffer) was loaded onto the column followed by 100 ml isocratic elution and a 500 ml linear gradient to 0.5 M NaCl. All protein eluted in a single broad band prior to the phenol red. The protein fractions were combined, dialyzed against 20 mM Tris, 20 mM NaCl, pH 8.0, and fractionated by Mono QTM anion exchange chromatography, and two major bands of protein were observed. To accommodate the increased scale of this preparation (300 ml) multiple anion exchange runs were used to fractionate all the DEAE-cellulose-processed protein.

All identical fractions from the various runs were combined, and an aliquot of each was fractionated on a size exclusion column, 7.5 x 300 mm ("G3000 SW TSK", Toyo Soda, Japan), isocratically eluted with 10 mM sodium phosphate, 50 mM sodium sulfate, pH 6.7. The anion exchange fractions containing 55-75 kDa protein were combined and subjected to fractionation on the size exclusion column, collecting that which eluted between 7.5 and 9.5 ml in two 1.0 ml fractions, representing the ascending and descending sides of the 60-70 kDa peak. The procedure was repeated 108 times using an automatic sampler.

The two pools were dialyzed against water and lyophilized. The resultant protein powder was dissolved in the Phenyl-Superose buffer described in EXAMPLE 1, and injected and eluted as described above. The albumin-like protein eluted in fractions 19-24, whereas bioactivity eluted in fractions 10-13. Each fraction was dialyzed against water and lyophilized. Electrophoretic analyses (SDS-polyacrylamide) and isoelectric focusing revealed that fractions 10-13 contained two proteins: one having a molecular weight of approximately 70 kDa and a pl of between about 5.2 and about 5.5, another having a molecular weight of approximately 55 kDa and a pl of less than about 4.0. The albumin-like peak (tubes 19-24) were (molecular weight approximately 65 kDa, pl about 4.1) very similar to genuine albumin.

Thus, the marker of the present invention can be biochemically distinguished from albumin.

## EXAMPLE 5

### Large Scale Purification of Cancer Marker

CEM cells were grown in 1 liter and 2 liter spinner culture as described in EXAMPLE 1. CEM cell-conditioned medium was collected 2-3 times a week, centrifuged to remove cells and other solids, and the supernatant solution was stored frozen at -20°C until use. 20 liters of cell-conditioned medium was concentrated ten-fold using a hollow fiber filter (Amicon LP-1™ pump and H1P10™ filter). NaCl was added (to a concentration of 1 M to 2 M to accelerate the removal of tightly bound phenol red), then the salt was exchanged for 0.1 mM HCl and the solution was concentrated to 100 ml, lyophilized, and stored at -20°C until used.

A chromatofocusing column ("Mono P™", Pharmacia) was prepared to fractionate the concentrated CEM cell-conditioned medium in the pH range of 6.3 to 4.0. The column was equilibrated with 5 mM bis-Tris, pH 6.3. Two ml samples of protein dissolved in and dialyzed against the bis-Tris buffer were injected into the column, and the column was eluted with buffer ("Polybuffer 74™", Pharmacia), diluted ten-fold, and titrated to pH 4.0. Two main peaks and several minor peaks, ten in all, were observed. RNA transport assays indicated biological activity in the two major peaks (peaks 2 and 4). Analytical acrylamide electrophoreses indicated that peak 2 contained a 70 kDa, pl 5.5 protein and that peak 4 contained 60-70 kDa, pl 5 proteins. Each peak also contained other proteins. Based on these observations the remaining concentrated CEM cell-conditioned medium was fractionated on a Mono P™ chromatofocusing column and combined into peak 2 and peak 4 pools for further purification.

Samples of peak 2 and peak 4 protein were chromatographed on a hydroxyapatite column ("HPHT™", Bio-Rad), which binds proteins ionically through the phosphate groups of the stationary phase. Bound proteins are eluted by an increasing gradient of phosphate.

The hydroxyapatite chromatography buffer A was 10 mM sodium phosphate, and buffer B was 300 mM sodium phosphate, both at pH 6.8. The column and sample were equilibrated with buffer A containing 0% buffer B, and a 16 ml linear gradient to 100% buffer B was developed at 0.5 ml/min. There was protein from each Mono P™ peak (2 and 4) which eluted at both low (50 mM) and high (250 mM) phosphate. RNA transport assays showed that the high phosphate-eluting peak contained the great preponderance of the biological activity. Since only a small amount of the activity resided in the low-phosphate-eluting fractions the high-eluting peaks were used for further analysis.

Hydroxyapatite-purified samples which demonstrated high RNA-transport activity were subjected to biochemical purity analysis using two-dimensional gel electrophoresis, according to the general method of O,Farrell et al., J. Biol. Chem., 250:4007 (1975), the disclosure of which is hereby incorporated by reference. In this procedure proteins are sequentially separated by two distinct methods, first by charge (isoelectric point) and then by molecular size, within a polymer matrix, allowing for the resolution of thousands of proteins if sensitive procedures for detection of the proteins are used.

Samples which had been dialyzed to remove most of the ionic species were first separated by charge using a pH 3 to 7 isoelectrofocusing agarose gel plate ("Isogel™", FMC Corp.) in an electrophoresis apparatus ("Phastsystem™", Pharmacia). Each sample was 4 μl of a 4 mg/ml solution. The lanes containing protein were cut from the agarose plate, rapidly dried using a hair dryer, rehydrated in a 10% (w/v) SDS denaturant solution, laid on a 10-15% (w/v) acrylamide gradient gel (Pharmacia), and electrophoresed in a second dimension for separation based on molecular size. The acrylamide gels were then stained for protein by a sensitive silver staining method using the reagents and procedures provided in the Bio-Rad (Richmond, CA) Silver Stain Kit.

Two-dimensional ("2D") gel electrophoretic analysis showed that the fractions which eluted at higher phosphate were similar whether originating from peak 2 or peak 4 of the Mono PTM chromatofocusing column step. Those from peak 2 consisted of a band of protein of about 70 kDa (migrating slightly slower than BSA in the SDS portion of the 2D gel) with a pI ranging between 5.1 and 5.7. There was a faint band (estimated by stain densities at less than 5% of the total silver-stained species) which was slightly less than bovine serum albumin ("BSA") molecular weight and with a lower pI than the major species (i.e., approximately 60 kDa and a pI of about 4.8).

The high-phosphate-eluting fraction derived from peak 4 showed a different distribution of proteins, having properties similar to those described in the above paragraph. From peak 4 the major peak (approximately 60% of the protein based on absorbance) was smaller than BSA with a pI below about 5.0 while the minor portion (approximately 40%) consisted of a molecular weight doublet of protein in which each band was slightly larger than BSA and ranged between about pI 5.0 and about pI 5.7.

Peak samples of each of the high-phosphate-eluting fractions were combined and analyzed by 2D electrophoresis. Only two major bands were found: 1) a band (which was the major portion of all the stained protein) that had a slightly greater molecular weight than BSA (70 kDa) and that had a minor component at slightly lower molecular weight yet still greater than albumin (the lower part of the doublet from peak 4), and 2) a band smaller than albumin (approximately 60 kDa), with a pI more acidic than the range of the pI's of the higher molecular weight band(s) (i.e., about 4.8).

## EXAMPLE 6

### Preparation of Monoclonal Antibodies to Marker

The following procedures were performed using the active fraction from the pooled peak 2 Mono PTM chromatofocusing column of EXAMPLE 5, which was additionally purified by elution from the HPHTTM hydroxyapatite column at high phosphate concentration. This marker fraction consisted of >95% pure protein (as judged by staining density) of approximately 70 kDa molecular weight with a pI ranging from about 5.1 to about 5.7.

Monoclonal antibodies were raised against marker in the following manner. Cells were extracted from the spleens and gently washed with RPMI medium, centrifuged, and resuspended in 10 ml of medium. Each fusion consisted of $1 \times 10^8$ spleen leukocytes and $2.5 \times 10^7$ NS-1 myeloma cells fused for 8 min. in the presence of neutralized polyethylene glycol. The cell suspension was allowed to stand overnight in serum-containing medium at 37° C in a tissue culture flask. Cells were then counted and distributed $7.5 \times 10^5$ per well in a 96 well tray.

IgG-producing clones were initially selected for expansion based on immunoblot analysis in which marker was electrophoresed followed by incubation with the hybridoma- conditioned medium overnight followed by incubation with phosphatase-labeled anti-mouse IgG. Clones were also screened for antibody specifically against the cancer marker in an enzyme linked immunosorbent assay ("ELISA") in which the marker (at 2 μg/well) was bound to ImmulonTM microtiter wells. After incubation with 50 μl of hybridoma medium the wells were incubated with phosphatase labeled second antibody.

Clones designated 30-07 and 30-24, two high IgG-producing lines which were among those which had survived the previous screenings, were tested by conventional dot blot ELISA analysis using serial dilutions of antibody (from 1:1 to 1:64) against adsorbed marker and also as a control against BSA. There was no absorbance in controls which contained no adsorbed antigen or in those in which the primary antibody was normal mouse serum or non-marker hybridoma conditioned medium. The highest absorbance values, at the 1:1 dilution, were 0.672 units (Clone 30-07) and 0.158 units (Clone 30-24). These values decreased serially to an absorbance value of 0.107 units (for Clone 30-07 at the 1:16 dilution) and 0.087 units (for Clone 30-24 at 1:32). There was no absorbance at any antibody dilution for the BSA controls.

Clone 30-07 was used to perform a dot blot ELISA assay with varying dilutions of marker. The highest concentration of marker protein was 2 μg per dot, and this was serially, two-fold diluted to 0.028 μg. Antibody diluted to 1:4 gave an absorbance value of 0.562 units at the highest concentration and decreased in proportion to the amount of antigen to the value of 0.053 units at the lowest concentration.

In repeated dot blot tests using human plasmas from control and cancer patients there was consistently greater absorbance from the cancer plasma than from controls (TABLE 3). Plasma was diluted 1:1 with PBS-Tween, and 50 µl was applied per dot. Antibody 30-07 (50 µl) was used.

TABLE 3

| ELISA Assay for Marker in Normal Control and Cancer Patient Plasma | | |
|---|---|---|
| Sample | Absorbance (S.D.) | Number of Assays |
| Blank | 0.036 (0.014) | 4 |
| Control Plasma | 0.029 (0.045) | 10 |
| Cancer Plasma | 0.126 (0.023) | 6 |
| Marker (purified) (1 µg protein) | 0.242 (0.053) | 2 |

As can be seen in TABLE 3, control plasmas are indistinguishable from blank solutions. The cancer plasmas are clearly different from the controls, and the result from the purified marker standard suggests that the cancer plasmas contain something less than half a microgram of the marker.

EXAMPLE 7

Confirmation of Specificity

The depletion of marker from human plasma was attempted using an immobilized monoclonal antibody of the present invention.

Thirty-two hybridoma cell-conditioned media were the sources of the mouse monoclonal antibodies used. They were selected using conventional electrophoretic and immunoblotting techniques based on their ability to combine with CEM cell proteins in the 60-70 kDa molecular weight range. Mouse immunoglobulin G (IgG) concentrations were determined using the Pandex™ (Baxter Labs) instrumentation. Briefly, polystyrene beads are coated with anti-mouse IgG antibodies, the beads are incubated with either known concentrations of mouse IgG or hybridoma medium of unknown concentration, and subsequently incubated with a second anti-mouse IgG antibody which has been coupled to a fluorescent molecule (fluorescein isothiocyanate). The amount of bound fluorescence is proportional to the amount of mouse IgG in the range of 90 pg/ml to 5 µg/ml.

Monoclonal antibody was immunochemically adsorbed onto polystyrene beads ("Pandex™") which were covalently linked to goat anti-mouse-IgG. Bead suspensions (0.25% (w/v)) contained 50 µg of anti-mouse-IgG per ml. 200 µl of bead suspension was incubated overnight at 4°C with 15 ml of each hybridoma-conditioned medium with continuous rocking. The beads were then separated from the conditioned medium by centrifugation and rinsed several times to remove passively adsorbed proteins. The final two rinses were in the 50 mM Tris, pH 7.5 buffer supplemented with 2.5 mM $MgCl_2$ and 25 mM KCl.

Monoclonal antibody-bead suspension (25 µl) was incubated with 100 µl of plasma test sample for 2 hr at 4°C with continuous rocking in order to remove marker from the plasma. The plasma solution was separated from the beads by centrifugation, and 100 µl of the supernatant solution was transferred into a tube for an RNA transport assay.

In a series of experiments the results of which are summarized below in TABLE 4, no decrease in RNA transport was observed in plasma incubated with 30 of the 32 media. However, two of the clones (31-4 and 31-5) reduced the RNA transport of human cancer plasma but not of rat cancer plasma. Clone 31-19 is included as typical of the 30 negative clones screened in these experiments.

TABLE 4

| Decrease in RNA Transport by Immobilized Antibodies | | |
| --- | --- | --- |
| Percent Decrease in RNA Transport (% of Control) | | |
| Antibody | Human Plasma | Rat Plasma |
| Blank | 0 % | 0 % |
| Clone 31-4 | 99 | 0 |
| Clone 31-5 | 104 | 0 |
| Clone 31-19 | 4 | - |
| Irrelevant Ab | 3 | - |

A deposit of the clone designated 31-05 has been made with the American Type Culture Collection, Rockville, MD, and assigned ATCC Accession No. HB 10251 (Applicant's designation 31-05, deposited October 10, 1989).

EXAMPLE 8

Cross-reactivity with Other Cancer-related Factors

The following procedure was performed in order to determine whether the factor described in the Senger '538 patent cited above was detectable by antibodies against the marker of the present invention.

Citrated blood was collected from five healthy individuals. Plasma was prepared by centrifugation, and a pool was prepared and stored in 2 ml aliquots at -70°C prior to the assay. The fractionation procedure described by Senger (US 4,725,538) to prepare a fraction enriched in factor described therein was followed. The enriched fraction liberated from the barium precipitate and resuspended in electrophoresis sample buffer containing citrate was subjected to electrophoresis through a 12.5% (w/v) acrylamide gel until the marker dye approached the bottom. The proteins were electrophoretically transblotted onto a nitrocellulose membrane.

After blocking residual sites with PBS-Tween the individual blots were then incubated with the undiluted conditioned medium containing antibodies 31-4 or 31-5, followed by washing and incubation with goat anti-mouse IgG conjugated with alkaline phosphatase. After additional washing the blots were incubated with 5-bromo-4-chloro-3-indolylphosphate, an alkaline phosphatase substrate (Kirkegaard and Perry Labs, Gaithersburg, MD). Only the positive control showed a positive purple stain, indicating that the detection chemistry was working. There was a negative response in the incubation of the hybridoma with the plasma pool, indicating that the monoclonal antibody was not reacting at a detectable level with a fraction that should contain the factor described by Senger. India ink staining showed that there were detectable amounts of protein present in the plasma pool.

**Claims**

1. A cancer marker characterized as a protein that
   (a) exhibits RNA-releasing activity,
   (b) is detectable in the serum of substantially all patients having either disseminated or localized tumors, and is not detectable by means of such an assay in normal human serum,
   (c) exhibits a molecular weight of approximately kDa, and
   (d) is of sufficient purity to allow the preparation of monoclonal antibodies specific for the marker.

2. Monoclonal antibodies specific for the cancer marker of claim 1.

3. Monoclonal antibodies according to claim 2 wherein the antibodies are the product of a hybridoma having ATCC Accession No. HB 10251.

4. A hybridoma capable of producing monoclonal antibodies according to claim 2.

5. A hybridoma according to claim 4 having ATCC Accession No. HB 10251.

6. Monoclonal antibodies prepared by a method that comprises the step of injecting a suitable host with the cancer marker of claim 1.

7. A method of purifying the cancer marker of claim 1 comprising the steps of

(a) concentrating a sample containing the marker to a point where further concentration is technically limited, and

(b) purifying the concentrated marker by chromatofocusing followed by hydroxyapatite chromatography.

8. A method for determining the presence or amount of the cancer marker of claim 1 comprising the steps of

(a) providing a quantity of monoclonal antibody specific for marker,

(b) contacting the monoclonal antibody with a sample suspected of containing marker under conditions suitable to allow an immunochemical reaction to occur between antibody and marker,

(c) detecting the extent of the immunochemical reaction, and

(d) correlating the extent of immunochemical reactions with the presence or amount of marker present in the sample.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X,D,Y | EP-A-0 145 373 (THE OHIO STATE UNIVERSITY RESEARCH FOUNDATION) <br> * claims * | 1-6,8,7 | C 07 K <br> 15/06 <br> C 12 P 21/08 <br> C 12 N 5/20 <br> C 07 K 3/28 <br> G 01 N 33/574 <br> G 01 N 33/577 |
| Y | ARCH. BIOCHEM. BIOPHYS. vol. 251, no. 1, 1986, New York, USA pages 385 - 392; H. TAKIKAWA et al.: "Binding of bile acids, oleic acid and organic anions by rat and human hepatic Z protein." <br> * page 386, left-hand column, lines 1 - 52 * | 7 | |
| Y | EP-A-0 244 999 (ROHM AND HAAS COMPANY) <br> * page 4, lines 18 - 45 * | 7 | |

| | TECHNICAL FIELDS SEARCHED (Int. Cl.5) |
|---|---|
| | C 07 K <br> C 12 P <br> C 12 N <br> G 01 N |

The present search report has been drawn up for all claims

| Place of search | Date of completion of search | Examiner |
|---|---|---|
| The Hague | 25 January 91 | NOOIJ F.J.M. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

 

& : member of the same patent family, corresponding document